(19) **Europäisches Patentamt**
**European Patent Office**
**Office européen des brevets**

(11) **EP 4 692 037 A1**

(12) **EUROPEAN PATENT APPLICATION**
published in accordance with Art. 153(4) EPC

(43) Date of publication:
**11.02.2026 Bulletin 2026/07**

(21) Application number: **24780483.4**

(22) Date of filing: **27.03.2024**

(51) International Patent Classification (IPC):
**C07C 43/23** (2006.01)

(52) Cooperative Patent Classification (CPC):
**C07C 43/23**

(86) International application number:
**PCT/JP2024/012302**

(87) International publication number:
**WO 2024/204381 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.03.2023 JP 2023056002**

(71) Applicant: **Kaneka Corporation**
**Osaka-shi, Osaka 530-8288 (JP)**

(72) Inventors:
• **HASHIMOTO, Takanori**
**Takasago-shi, Hyogo 676-8688 (JP)**
• **YAMAGUCHI, Takao**
**Takasago-shi, Hyogo 676-8688 (JP)**
• **TANIZAKI, Subaru**
**Takasago-shi, Hyogo 676-8688 (JP)**

(74) Representative: **Hoffmann Eitle**
**Patent- und Rechtsanwälte PartmbB**
**Arabellastraße 30**
**81925 München (DE)**

(54) **CRYSTAL AND PREPARATION OF REDUCED COENZYME Q10**

(57) Provided is a crystal of reduced coenzyme Q10, the crystal having excellent oxidation stability. The crystal of reduced coenzyme Q10 has a melting point of 52.0°C or more, as measured at a heating rate of 1°C/minute by differential scanning calorimetry (DSC), wherein the melting point is represented by a temperature at which a cumulative calorific value becomes 50%.

Fig. 1

The arrow denotes a temperature at which the area of the shadowed portion becomes 50% of the peak area

EP 4 692 037 A1

**Description**

Technical Field

The present invention relates to reduced coenzyme Q10 useful as a material for a supplement, and particularly to a crystal of the coenzyme and to a formulation of the crystal.

Background Art

Coenzyme Q is an essential component widely distributed in living organisms from bacteria to mammals, and is known as a member of the mitochondrial electron transfer system in cells in the living organisms. In humans, the major form of coenzyme Q is coenzyme Q10, which has 10 repeating side chains of coenzyme Q. In the living body, approximately 40% to 90% of the coenzyme Q10 is usually present in reduced form.
It has been reported that coenzyme Q10 has a wide range of physiologically active effects, such as activation of energy production by mitochondrial activation, activation of cardiac function, an effect of stabilizing cell membranes, and an effect of protecting cells by antioxidant activity. The coenzyme is utilized as a medicine or a material for a supplement.
Conventionally, coenzyme Q10 used for a supplement or the like has often been oxidized coenzyme Q10, but reduced coenzyme Q10 is being more widely utilized because reduced coenzyme Q10 has higher oral absorbability than oxidized coenzyme Q10 (Patent Literature 1).
On the other hand, reduced coenzyme Q10 is easily oxidized by oxygen in the air. A supplement containing reduced coenzyme Q10 as a main component is mainly in the form of a soft capsule, such as a gelatin capsule packed with fat containing reduced coenzyme Q10.
For application in a tablet, a hard capsule, and the like, reduced coenzyme Q10 needs to be increased in oxidation stability, and various studies are being made. For example, Patent Literature 2 discloses that a solid composition containing reduced coenzyme Q10 is coated with a coating medium such as shellac, gelatin, or gum arabic to be thereby stabilized.
In addition, it has been verified in recent years that crystal polymorphism exists among the crystals of reduced coenzyme Q10. A crystal (Form II crystal) having more excellent oxidation stability than a conventional type of crystal (Form I crystal) has been discovered (Patent Literature 3).

Citation List

Patent Literature

Patent Literature 1: WO 98/07417
Patent Literature 2: WO 2008/129980
Patent Literature 3: WO 2012/176842

Summary of Invention

Technical Problem

When the present inventors produced a tablet-type of supplement experimentally, using a reduced coenzyme Q10 Form II crystal, the supplement was inhibited from being oxidized during processing, and a formulation exhibiting a high content of reduced coenzyme Q10 was obtained. On the other hand, it has been ascertained that further enhancement of the oxidation stability is desired, taking into account the distribution and storage of the formulation.
In view of the above-described problem, the present invention is to provide a reduced coenzyme Q10 crystal and a formulation having excellent oxidation stability.

Solution to Problem

The present inventors have made studies vigorously, and as a result, have come to complete each below-described form of the present invention through the discovery that a reduced coenzyme Q10 crystal called a Form II crystal has a range of melting points depending on the manufacturing condition or the like, and that a crystal having a higher melting point exhibits higher oxidation stability.

[1] A crystal of reduced coenzyme Q10, having a melting point of 52.0°C or more, as measured at a heating rate of 1°C/minute by differential scanning calorimetry (DSC), wherein the melting point is represented by a temperature at which a cumulative calorific value becomes 50%.

[2] The crystal according to [1], wherein an oxidizing rate of the crystal, as represented by the following Formula, is 0.40 or less when the crystal is stored in a condition of 40°C and 75% RH for 1 month.

[3] The crystal according to [1] or [2], which exhibits characteristic peaks at diffraction angles (2θ ± 0.2°) of 11.5°, 18.2°, 19.3°, 22.3°, 23.0°, and 33.3° in powder X-ray (Cu-Kα) diffractometry.

[4] A solid formulation including a crystal of reduced coenzyme Q10,
wherein, when differential scanning calorimetry (DSC) is performed on 30 ± 1 mg of the formulation at a heating rate of 1°C/minute, a melting point (y) (°C) represented by a temperature at which a cumulative calorific value becomes 50% and an amount (x) (mg) of the crystal of reduced coenzyme Q10 contained in the 30 ± 1 mg of the formulation satisfy the following Formula (1).

$$(1): y \geq 0.1762x + 51.327$$

The present specification encompasses the disclosure of Japanese Patent Application No. 2023-056002 that serves as a basis for the priority of the present application.

Advantageous Effects of Invention

The present invention provides a crystal of reduced coenzyme Q10 and a formulation thereof that have excellent oxidation stability, and can be used for various forms of supplements.

Brief Description of Drawing

[Figure 1] Figure 1 schematically illustrates a temperature at which a cumulative calorific value becomes 50%.
[Figure 2] Figure 2 is a graph in which an approximate straight line indicates the relationship between the melting point of a reduced coenzyme Q10 crystal and the oxidizing rate.

Description of Embodiments

The present invention will be described in detail below.
As described above, the reduced coenzyme Q10 encompasses two forms of crystal polymorphisms, i.e., Form I and Form II. Specifically, the crystalline form of reduced coenzyme Q10, having a melting point of approximately 48°C and showing characteristic peaks at diffraction angles (2θ ± 0.2°) of 3.1°, 18.7°, 19.0°, 20.2°, and 23.0° in powder X-ray (Cu-Kα) diffractometry, is called a Form I crystal. The crystalline form of reduced coenzyme Q10, having a melting point of approximately 52°C and showing characteristic peaks at diffraction angles (2θ ± 0.2°) of 11.5°, 18.2°, 19.3°, 22.3°, 23.0°, and 33.3° in powder X-ray (Cu-Kα) diffractometry, is called a Form II crystal.
The present inventors have made studies, and as a result, have verified that the melting point of a crystal called a Form II crystal varies in a certain range, depending on the manufacturing condition and the like. The crystal having a higher melting point tends to have higher oxidation stability.
Specifically, it has been verified that a crystal having a melting point of 52.0°C or more (as measured by differential scanning calorimetry (DSC) performed at a heating rate of 1°C/minute, wherein the melting point is represented by a temperature at which a cumulative calorific value becomes 50%) exhibits a noticeable increase in oxidation stability, compared with a crystal having a melting point lower than the temperature, and that the oxidizing rate represented by the following Formula is 0.40 or less when the crystal is stored in a condition of 40°C and 75% RH for 1 month. In the present disclosure, the melting point of a crystal means a melting point measured by differential scanning calorimetry (DSC) performed on a sample in an amount of 8 ± 1 mg.

Oxidizing rate (%/day) = (100 - relative QH ratio after storage)/number of days of storage

Here, the "QH ratio" means the ratio of the amount of the reduced coenzyme Q10 to the total amount of the coenzyme Q10 present in the crystal, and is a value calculated in accordance with the following formula.

QH ratio (%) = reduced coenzyme Q10 content/(oxidized coenzyme Q10 content + reduced coenzyme Q10 content)

The "relative QH ratio after storage" is a value obtained by relativizing the QH ratio after storage on the basis of each of the QH ratios measured before the start of storage and after storage, assuming that the QH ratio before storage is 100. Specifically, the ratio is calculated in accordance with the following formula

Relative QH ratio (%) after storage = QH ratio after storage/QH ratio before start of storage $\times$ 100

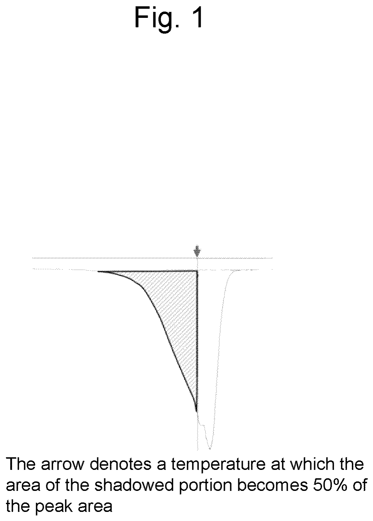

In addition, the "temperature at which a cumulative calorific value becomes 50%" defined as a melting point of a crystal in the present invention means a temperature at which the area of a portion surrounded by an endothermic peak and a baseline first becomes 50% of the peak area as the area of the portion is integrated at intervals of 0.02°C from the low-temperature side in a DSC chart. The temperature is schematically illustrated by the temperature denoted by the arrow in Figure 1.

As described above, the melting point of a crystal according to the present invention is a temperature at which a cumulative calorific value becomes 50%, as measured at a heating rate of 1°C/minute, and is 52.0°C or more. The melting point is more preferably 52.2°C or more, 52.3°C or more, 52.4°C or more, 52.5°C or more, or 52.6°C or more.

The upper limit of the melting point of a crystal according to the present invention is not particularly limited, as long as the crystal can be handled and stored in the form of a solid without any problem. A temperature (that is a melting point of a crystal according to the present invention, and is a temperature at which a cumulative calorific value becomes 50%, as measured at a heating rate of 1°C/minute) is, for example, 54.5°C or less, preferably 54.0°C or less, more preferably 53.5°C or less, still more preferably 53.0°C or less.

When a crystal according to the present invention is stored in a condition of 40°C and 75% RH for 1 month, the oxidizing rate of the crystal is 0.40 or less as described above, more preferably 0.35 or less, 0.30 or less, 0.28 or less, 0.26 or less, 0.24 or less, or 0.23 or less.

A crystal that is a crystal of reduced coenzyme Q10 and exhibits the above-described melting point and/or oxidizing rate belongs to a crystal according to the present invention, and is preferably a crystal that exhibits an XRD diffraction pattern characteristic of a Form II crystal. That is, a crystal according to the present invention is preferably a crystal that exhibits characteristic peaks at diffraction angles (2θ ± 0.2°) of 11.5°, 18.2°, 19.3°, 22.3°, 23.0°, and 33.3° in powder X-ray (Cu-Kα) diffractometry.

A crystal according to the present invention can be efficiently obtained using a step of crystallizing reduced coenzyme Q10 into a Form II crystal (a cooling crystallization method), the step including, for example, an operation incorporated therein, in which operation the temperature in a crystallization vessel is raised temporarily to dissolve part of a crystal precipitated.

Coenzyme Q10 can be crystallized into a Form II crystal, for example, by adding a Form II crystal preliminarily obtained as a seed crystal to an ethanol solution containing reduced coenzyme Q10, and cooling the resulting mixture. The cooling rate, if too high, will undesirably cause a Form I crystal to mingle in the solution, and thus, the cooling is preferably performed by controlling the cooling rate suitably.

As a specific condition, the condition disclosed in WO 2022/202215 can be applied. For example, a seed crystal is added at 37°C to an ethanol solution containing reduced coenzyme Q10. Then, the cooling rate is controlled in such a manner that the rate of change of turbidity is 45 FTU/min or less, during the period from at least 1,000 FTU to 10,000 FTU, for which turbidity, formazine turbidity (FTU/min) in a crystallization vessel is used as an index. Whereby, a Form I crystal is inhibited from mingling in, thus enabling a high-quality Form II crystal to be obtained. In this regard, the rate of change of turbidity is also referred to as a turbidity-change rate.

In one method of obtaining a crystal according to the present invention, an operation for dissolving part of a crystal precipitated is added to the above-described crystallization step. Specifically, part of a crystal precipitated is dissolved by raising the temperature in the crystallization vessel at a point in time after the rising of the turbidity in a crystallization vessel, the precipitation of the crystal is verified successfully. "Dissolving part of a crystal" can be detected owing to a decrease in the turbidity (which does not become 0, however). Dissolving part of a crystal is followed by decreasing the temperature in the crystallization vessel again to restart cooling crystallization.

The point in time when the temperature rise starts is not particularly limited, as long as precipitated crystals are present in the crystallization vessel. From the viewpoint of avoiding all of the precipitated crystals to be dissolved by raising the temperature, it is desirable for a certain amount of crystals to be present. In addition, the partial dissolution is verified by a change in turbidity, it is desirable that the dissolution is within the measurement range of the turbidimeter (below the upper limit of measurement). In general, it is preferable that the point in time is in the period during which the turbidity, when used as an index, is from 8000 to 10000 FTU, or in the period during which the temperature in the vessel, when used as an index, is from 33 to 37°C.

The degree of the temperature rise is not particularly limited, as long as all the precipitated crystal is dissolved. When a change in turbidity is used as an index, and on the premise that the turbidity does not become 0, the degree is preferably such that the range of decrease in turbidity is approximately from 350 to 8000 FTU. When the temperature in the vessel is used as an index, the degree is such that the range of the temperature rise is approximately from 0.5 to 6.5°C.

In this regard, the heating operation (that dissolves part of a crystal precipitated) is usually performed once, or may be performed twice or more times, if desired.

A crystal of reduced coenzyme Q10 is precipitated through the above-described step, and, in the same manner as a conventional crystal of reduced coenzyme Q10, undergoes solid-liquid separation by filtration or centrifugation, is then dried, isolated, and collected in the form of a crystal according to the present invention. As to the methods and conditions for the solid-liquid separation and drying, known conditions for methods for solid-liquid separation and drying of a reduced coenzyme Q10 crystal can be applied.

Next, a formulation according to the present invention will be described. One embodiment of a formulation according to the present invention is a solid formulation containing a crystal of reduced coenzyme Q10. When differential scanning calorimetry (DSC) is performed on $30 \pm 1$ mg of the formulation at a heating rate of 1°C/minute, a melting point (y) (°C) represented by a temperature at which a cumulative calorific value becomes 50% and an amount (x) (mg) of the crystal of reduced coenzyme Q10 contained in the $30 \pm 1$ mg of the formulation satisfy the following Formula (1).

$$(1): y \geq 0.1762x + 51.327$$

A study by the present inventors has discovered, through a plurality of experiments, that a formulation containing a reduced coenzyme Q10 crystal, compared with a reduced coenzyme Q10 crystal alone, tends to decrease the melting point of the reduced coenzyme Q10 crystal, as observed through DSC, but a formulation that satisfies the Formula (1) excels in the oxidation stability of reduced coenzyme Q10, and thus, can be used for various forms of supplements and the like. The formulation that satisfies the Formula (1) can be obtained, for example, by using, as a reduced coenzyme Q10 crystal, a reduced coenzyme Q10 crystal according to the present invention. That is, another embodiment of a formulation according to the present invention is, for example, a solid formulation containing a reduced coenzyme Q10 crystal according to the present invention.

The melting point (y) (°C) of the formulation is not particularly limited, and is, for example, 51.0°C or more, more preferably 51.2°C or more, 51.3°C or more, 51.4°C or more, 51.5°C or more, or 51.6°C or more. In addition, the melting point (y) (°C) of the formulation is, for example, 53.5°C or less, preferably 53.0°C or less, more preferably 52.5°C or less, still more preferably 52.0°C or less.

In addition, when an XRD diffraction pattern of the formulation is observed, the reduced coenzyme Q10 crystal in the formulation preferably exhibits a pattern characteristic of a Form II crystal. That is, the reduced coenzyme Q10 crystal in the formulation preferably exhibits characteristic peaks at diffraction angles ($2\theta \pm 0.2°$) of 11.5°, 18.2°, 19.3°, 22.3°, 23.0°, and 33.3° in powder X-ray (Cu-K$\alpha$) diffractometry.

A formulation according to the present invention contains a component other than a crystal of reduced coenzyme Q10. The component other than the crystal of reduced coenzyme Q10 is not particularly limited. The component other than the crystal of reduced coenzyme Q10 may be selected suitably in accordance with, for example, the application of the formulation. In addition, a method of producing a formulation according to the present invention is not particularly limited, and the formulation may be produced using a known method in accordance with, for example, the application of a formulation.

Examples of the component other than the crystal of reduced coenzyme Q10 include lubricants and base materials.

Examples of the lubricants include magnesium stearate, calcium stearate, and sucrose fatty acid esters. Examples of the base materials include microcrystalline cellulose, crystalline cellulose, maltitol, starch, HPC (hydroxypropyl cellulose), and palatinose.

The amount of the reduced coenzyme Q10 crystal contained in the formulation is not particularly limited, and can be suitably selected in accordance with the application or the like to the extent of causing no problem in the processing of the formulation. The amount of the reduced coenzyme Q10 crystal contained in the formulation is usually 1 wt% or more, preferably 2 wt% or more, 5 wt% or more, or 10 wt% or more. The amount of the reduced coenzyme Q10 crystal contained in the formulation is usually 30 wt% or less, preferably 25 wt% or less, 20 wt% or less, 15 wt% or less, or 10 wt% or less.

Examples

The present embodiment will be described below with reference to Examples, and the present invention is not limited to these Examples. In addition, reduced coenzyme Q10 is referred to as QH in Examples.

(Comparative Example 1) Crystal obtained through usual cooling crystallization (with no midway temperature rise)

To a 36°C solution (QH reaction solution) in which QH was dissolved at 10 wt% in ethanol having a purity of 99.5 wt% or more, a Form II QH crystal separately prepared as a seed crystal was added at 2 wt% with respect to the weight of the QH dissolved. The precipitating rate of the crystal was controlled at a turbidity-change rate of 15.8 FTU/min until the turbidity reached 10000 FTU. After 10000 FTU was reached, the cooling was controlled at 1°C/hr until 25°C and at 10°C/hr until 1°C. The QH crystal in the resulting reaction solution was obtained through vacuum filtration, and then dried in vacuo for 24 hours to prepare a dried QH crystal.

The resulting crystal underwent a DSC analysis under the following conditions, and the melting point was measured. As a result, the melting-point temperature was 51.4°C (the temperature at which the cumulative calorific value became 50% was regarded as the melting-point temperature).

(DSC Measurement Conditions)

Device: DSC200 (manufactured by Hitachi High-Tech Corporation)
Sample container: Aluminum pan & cover (GCA-0052)
Heating rate: 1°C/min
Amount of sample: 8 ± 1 mg
Granularity of sample: 53 to 74 $\mu$m

(Comparative Example 2) Crystal obtained through usual cooling crystallization (with no midway temperature rise)

A QH crystal was prepared in the same manner as in Comparative Example 1 except that the seed crystal at 8 wt% with respect to the weight of the QH dissolved was added to the 37°C QH reaction solution.

The DSC melting point of the resulting QH crystal was measured under the same conditions as in Comparative Example 1, and as a result, the melting-point temperature was 51.8°C.

(Example 1) Crystal obtained through cooling crystallization with midway temperature rise

At a point in time when the temperature of a reaction solution (QH reaction solution) in which QH was dissolved at 10 wt% in ethanol having a purity of 99.5 wt% or more was 37°C, a Form II QH crystal as a seed crystal was added at 8 wt% with respect to the weight of the QH dissolved. The precipitating rate of the crystal was controlled at a turbidity-change rate of 15.8 FTU/min until the turbidity reached 10000 FTU. After 10000 FTU was reached, the temperature was raised to 38.5°C. A decrease in turbidity was verified, and then, again on the basis of the value of turbidity, a crystal was precipitated at a rate of 25 FTU/min until 10000 FTU. After 10000 FTU was reached, the resulting crystal was cooled at 1°C/hr until 25°C and at 10°C/hr until 1°C. The QH crystal in the resulting reaction solution was obtained through vacuum filtration, and then dried in vacuo for 24 hours to prepare a dried QH crystal.

The DSC melting point of the resulting QH crystal was measured under the same conditions as in Comparative Example 1, and as a result, the melting-point temperature was 52.4°C.

(Example 2) Crystal obtained through cooling crystallization with midway temperature rise

A QH crystal was prepared in the same manner as in Example 1 except that the crystalprecipitating rate was changed to 15 FTU/min, in which the rate was used until the turbidity reached 10000 FTU again after a rise in temperature and a decrease in turbidity were ascertained.

The DSC melting point of the resulting QH crystal was measured under the same conditions as in Comparative Example 1, and as a result, the melting-point temperature was 52.6°C.

(Example 3) Crystal obtained through cooling crystallization with midway temperature rise

A QH crystal was prepared in the same manner as in Example 1 except that the crystalprecipitating rate was changed to 15 FTU/min, in which the rate was used until the turbidity reached 10000 FTU again after a rise in temperature and a decrease in turbidity were ascertained.

The DSC melting point of the resulting QH crystal was measured under the same conditions as in Comparative Example 1, and as a result, the melting-point temperature was 52.6°C.

(Example 4 and Comparative Example 3)

The QH crystals prepared in Examples 1 to 3 and Comparative Examples 1 and 2 above were stored at 40°C and 75% RH in a thermostatic chamber for approximately 1 month. Before and after the storage, the reduced coenzyme Q10 content and the oxidized coenzyme Q10 content of the crystal were measured by HPLC (high performance liquid chromatography). On the basis of the measurement results, the below-described QH ratio, relative QH ratio after storage, and oxidizing rate were calculated.

(HPLC conditions)

Column: AS12S05-1546WT (manufactured by YMC Co., Ltd.) 150 mm (in length) and 4.6 mm (in inner diameter)
Mobile phase: $C_2H_3N$:$CH_3OH$ = 1:9 (v:v)
Detection wavelength: 290 nm
Flow rate: 1 ml/min

The "QH ratio" means the ratio of the amount of the reduced coenzyme Q10 to the total amount of the coenzyme Q10 present in the crystal, and is calculated in accordance with the following formula.

QH ratio (%) = reduced coenzyme Q10 content/(oxidized coenzyme Q10 content + reduced coenzyme Q10 content)

The "relative QH ratio after storage" is a value obtained by relativizing the QH ratio after storage on the basis of each of the QH ratios measured before the start of storage and after storage, assuming that the QH ratio before storage is 100. The relative QH ratio after storage is calculated in accordance with the following formula.

Relative QH ratio (%) after storage = QH ratio after storage/QH ratio before start of storage $\times$ 100

The "oxidizing rate" is a value obtained by dividing a change (amount of decrease) in the relative QH ratio in the period of storage by the number of days of storage, and is calculated in accordance with the following formula.

Oxidizing rate (%/day) = (100 - relative QH ratio after storage)/number of days of storage

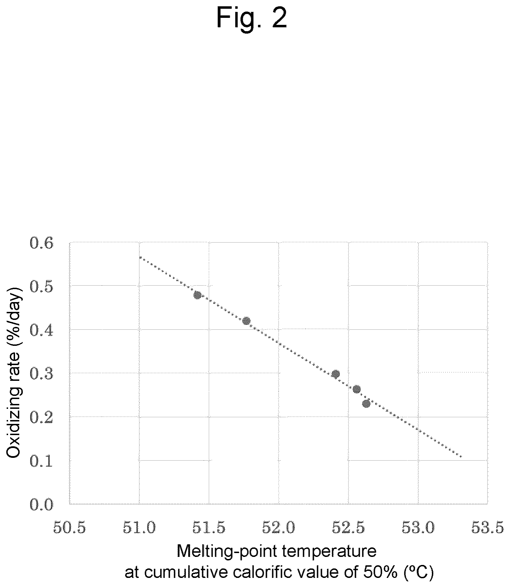

The results of measurements of the crystals prepared in Examples and Comparative Examples are summarized in the following Table 1. In addition, the approximate straight line of the melting point and the oxidizing rate, the line prepared from the results in Table 1, is shown in Figure 2.

[Table 1]

| QH crystal | Melting-point temperature at cumulative calorific value of 50% (°C) | Relative QH ratio (%) | Oxidizing rate (%/day) |
|---|---|---|---|
| Comparative Example 1 | 51.4 | 86.1 (when stored for 29 days) | 0.48 |
| Comparative Example 2 | 51.8 | 88.2 (when stored for 28 days) | 0.42 |
| Example 1 | 52.4 | 91.6 (when stored for 28 days) | 0.30 |
| Example 2 | 52.6 | 92.6 (when stored for 28 days) | 0.26 |
| Example 3 | 52.6 | 93.5 (when stored for 28 days) | 0.23 |

It is understood from Table 1 and Figure 2 that the melting point and oxidizing rate of the QH Form II crystal have high correlation therebetween, and that, among the Form II crystals, a crystal having a melting point of 52.0°C or more has particularly excellent oxidation stability, in which the melting point is represented by a temperature at which a cumulative calorific value becomes 50%.

(Example 5)

The QH crystal (having a melting-point temperature of 52.4°C) obtained in Example 1, magnesium stearate, and microcrystalline cellulose were mixed at a ratio shown in the following Table 2 to obtain a powdery formulation.

The resulting formulation underwent a DSC analysis under the below-described conditions, and the melting point was measured to determine the melting-point temperature (the temperature at which the cumulative calorific value became 50% was regarded as the melting-point temperature). Each melting-point temperature is shown in the following Table 2.

(DSC Measurement Conditions)

Device: DSC200 (manufactured by Hitachi High-Tech Corporation)
Sample container: Aluminum pan & cover (CVB-0008)
Heating rate: 1°C/min
Amount of sample: 30 $\pm$ 1 mg
Measurement temperature: 35°C to 60°C

[Table 2]

| | QH crystal | | Mg stearate | | Microcrystalline cellulose | | Melting-point temperature at cumulative calorific value of 50% |
|---|---|---|---|---|---|---|---|
| | (wt%) | (mg) | (wt%) | (mg) | (wt%) | (mg) | (°C) |
| Powder containing QH at 1 wt% | 1 | 3 | 2 | 6 | 97 | 291 | 51.38 |
| Powder containing QH at 2.5 wt% | 2.5 | 7.5 | 2 | 6 | 95.5 | 286.5 | 51.62 |
| Powder containing QH at 5 wt% | 5 | 15 | 2 | 6 | 93 | 279 | 51.62 |
| Powder containing QH at 7.5 wt% | 7.5 | 22.5 | 2 | 6 | 90.5 | 271.5 | 51.88 |
| Powder containing QH at 10 wt% | 10 | 30 | 2 | 6 | 88 | 264 | 51.86 |

(Example 6)

5 mg of the QH crystal (having the melting-point temperature of 52.4°C) obtained in Example 1, 2 mg of magnesium stearate, and 93 mg of each base material shown in the following Table 3 were mixed to obtain a powdery formulation (powder containing QH at 5 wt%). In this regard, HPC in Table 3 means hydroxypropyl cellulose.

The resulting formulation underwent a DSC analysis under the below-described conditions, and the melting point was measured to determine the melting-point temperature (the temperature at which the cumulative calorific value became 50% was regarded as the melting-point temperature). Each melting-point temperature is shown in the following Table 3.

(DSC Measurement Conditions)

Device: DSC200 (manufactured by Hitachi High-Tech Corporation)
Sample container: Aluminum pan & cover (CVB-0008)
Heating rate: 1°C/min

Amount of sample: 30 $\pm$ 1 mg
Measurement temperature: 35°C to 60°C

[Table 3]

| Base material | Amount of DSC sample | Melting-point temperature at cumulative calorific value of 50% |
|---|---|---|
| | (mg) | (°C) |
| Crystalline cellulose | 29.8 | 51.6 |
| Maltitol | 30.9 | 51.9 |
| Starch | 30.5 | 51.7 |
| HPC | 30.8 | 51.9 |

The upper limits and/or lower limits of the ranges of values herein described can be arbitrarily combined to define a preferable range. For example, any upper limit and any lower limit of the ranges of values can be combined to define a preferable range. Any upper limits of the ranges of values can be combined to define a preferable range. In addition, any lower limits of the ranges of values can be combined to define a preferable range.

It should be understood that expressions in the singular herein include the plural, unless otherwise specified. Accordingly, the singular articles (for example, "a", "an", and "the" in English) include plural references unless otherwise specified.

All publications, patents, and patent applications cited herein are incorporated herein by reference in their entirety.

**Claims**

1.  A crystal of reduced coenzyme Q10, having a melting point of 52.0°C or more, as measured at a heating rate of 1°C/minute by differential scanning calorimetry (DSC), wherein the melting point is represented by a temperature at which a cumulative calorific value becomes 50%.

2.  The crystal according to claim 1, wherein an oxidizing rate of the crystal, as represented by the following Formula, is 0.40 or less when the crystal is stored in a condition of 40°C and 75% RH for 1 month;

    Oxidizing rate (%/day) = (100 - relative QH ratio after storage)/number of days of storage.

3.  The crystal according to claim 1 or 2, which exhibits characteristic peaks at diffraction angles (2θ $\pm$ 0.2°) of 11.5°, 18.2°, 19.3°, 22.3°, 23.0°, and 33.3° in powder X-ray (Cu-Kα) diffractometry.

4.  A solid formulation comprising a crystal of reduced coenzyme Q10,
    wherein, when differential scanning calorimetry (DSC) is performed on 30 $\pm$ 1 mg of a formulation at a heating rate of 1°C/minute, a melting point (y) (°C) represented by a temperature at which a cumulative calorific value becomes 50% and an amount (x) (mg) of the crystal of reduced coenzyme Q10 contained in the 30 $\pm$ 1 mg of the formulation satisfy the following Formula (1).

$$(1):\ y \geq 0.1762x + 51.327$$

# Fig. 1

The arrow denotes a temperature at which the area of the shadowed portion becomes 50% of the peak area

# Fig. 2

## INTERNATIONAL SEARCH REPORT

International application No.

**PCT/JP2024/012302**

| A. CLASSIFICATION OF SUBJECT MATTER |
|---|
| *C07C 43/23*(2006.01)i<br>FI: C07C43/23 C |

According to International Patent Classification (IPC) or to both national classification and IPC

| B. FIELDS SEARCHED |
|---|

Minimum documentation searched (classification system followed by classification symbols)

C07C43/23

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JST 7580/JSTChina (JDreamIII), CAplus/REGISTRY (STN)

| C. DOCUMENTS CONSIDERED TO BE RELEVANT |
|---|

| Category* | Citation of document, with indication, where appropriate, of the relevant passages | Relevant to claim No. |
|---|---|---|
| X | WO 2012/176842 A1 (KANEKA CORP.) 27 December 2012 (2012-12-27)<br>claims, paragraphs [0022], [0055], [0067] | 1-4 |
| A | JP 2021-514370 A (CENTER FOR INTELLIGENT RESEARCH IN CRYSTAL ENGINEERING, S. L.) 10 June 2021 (2021-06-10)<br>entire text, all drawings | 1-4 |

☐ Further documents are listed in the continuation of Box C.    ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **03 June 2024** | **18 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

INTERNATIONAL SEARCH REPORT
Information on patent family members

International application No.

**PCT/JP2024/012302**

| Patent document cited in search report | | | Publication date (day/month/year) | Patent family member(s) | | | Publication date (day/month/year) |
|---|---|---|---|---|---|---|---|
| WO | 2012/176842 | A1 | 27 December 2012 | US | 2014/0120073 | A1 | |
| | | | | claims, paragraphs [0030], [0064], [0097] | | | |
| | | | | EP | 2725004 | A1 | |
| | | | | CN | 103635452 | A | |
| | | | | KR | 10-2014-0061350 | A | |
| JP | 2021-514370 | A | 10 June 2021 | US | 2020/0385327 | A1 | |
| | | | | EP | 3755683 | A1 | |
| | | | | CN | 112041294 | A | |

Form PCT/ISA/210 (patent family annex) (July 2022)

**REFERENCES CITED IN THE DESCRIPTION**

**Patent documents cited in the description**

- WO 9807417 A
- WO 2008129980 A
- WO 2012176842 A
- JP 2023056002 A
- WO 2022202215 A